# EUROPEAN PATENT SPECIFICATION

(11) **EP 2 685 937 B1**
(45) Date of publication and mention of the grant of the patent: **25.11.2015**
(21) Application number: 11710459.6
(22) Date of filing: 18.03.2011
(51) Int. Cl.: A61F 2/38

(54) **A FLOATING INSERT FOR A KNEE JOINT PROSTHESIS AND A KNEE JOINT PROSTHESIS INCLUDING SAME**
SCHWIMMENDER EINSATZ FÜR EINE KNIEGELENKPROTHESE UND EINE KNIEGELENKPROTHESE DAMIT
INSERT FLOTTANT POUR PROTHÈSE DU GENOU ET PROTHÈSE DU GENOU LE COMPRENANT

(43) Date of publication of application: 22.01.2014
(73) Proprietor: Smith & Nephew Orthopaedics AG, 6343 Rotkreuz (CH)
(72) Inventor: HOCHULI, Patrick, CH-5057 Reitnau (CH)
(74) Representative: Smith & Nephew
(86) International application number: PCT/EP2011/054140
(87) International publication number: WO 2012/126497

(56) References cited:
- EP-A1- 1 327 424
- EP-A1- 1 974 694
- EP-A1- 1 974 696
- WO-A2-03/094782
- WO-A2-2010/004342

## Description

The present invention relates to a floating insert for a knee joint prosthesis and to a knee joint prosthesis including such an insert. The closest prior art is WO 2010/004342 A2, which defines the preamble of claim 1.

Many varying prosthetic devices are available for knee joint replacement. These include bicondylar prosthetic devices, where both condyles of the knee are replaced, and unicondylar prosthetic devices wherein only one of the knee condyles is replaced. In such a procedure, tibial components are used to replace either or both of the medial and lateral condyles of the patient. A single, total femoral component or two partial femoral components may then be used to cooperate with the tibial component or components. However, unicondylar prostheses have to be precisely installed otherwise they are prone to failure. The Oxford unicondylar knee system was introduced to overcome this problem. In this system, a floating insert is located between a tibial component in the form of a tibial tray and the femoral component so that the insert can move with respect to both the tibial tray and the femoral component. This reduces the degree of precision required to produce a satisfactory knee joint. However, as the insert floats relative to the tibial component, it is free to rotate but excessive rotation of is undesirable.

One aim of the present invention is to provide a floating insert for use in a unicondylar knee joint prosthesis that is adapted to limit rotation of the insert relative to a tibial tray of the prosthesis.

A further aim is to provide a knee joint prosthesis including the aforementioned floating insert.

According to a first aspect of the present invention there is provided a floating insert for a unicondylar knee joint prosthesis as defined in claim 1.

Preferably, an angle between the intercondylar side and a posterior side of the insert is equal to or less than 90°.

Preferably also, corners between a superficial side of the insert and its posterior and anterior sides are rounded.

Preferably also, a surface of the tibial side of the insert is smooth.

Preferably also, an anterior-posterior profile of the insert is substantially wedge-shaped with an anterior height greater than a posterior height.

Preferably also, in the sagittal plane the difference in height between a deepest point of the femoral side and a posterior edge of the femoral side is no greater than 3 mm and is preferably of the order of 2mm

Preferably also, in the sagittal plane the difference in height between a deepest point of the femoral side and an anterior edge of the femoral side is between 2mm and 6 mm inclusive and is preferably of the order of 4 mm.

Preferably also, the insert is comprised of one or a combination of an ultra high molecular weight polyethylene, a ceramic or oxidized zirconium.

According to a second aspect of the present invention there is provided a unicondylar knee joint prosthesis comprising a femoral component, a tibial tray, and a floating insert, the floating insert being in accordance with the first aspect of die present invention.

Preferably, the tibial tray comprises a platform including a proximal bearing surface and a distal fixation surface, wherein the proximal bearing surface is smooth.

Preferably also, the platform of the tibial tray defines a wall that projects in a proximal direction at a intercondylar edge of the proximal bearing surface. Advantageously, the wall extends proximally, in use, in a sagittal plane.

Preferably also, said outer surface of the femoral component defines two regions for flexion rotation in a sagittal plane of which a first region has a first radius of curvature and a second region has a second radius of curvature, the first radius of curvature being larger than the second radius of curvature, and the surface of the femoral side of the insert has substantially the same radius of curvature in the sagittal plane as the first radius of curvature of the femoral component.

Preferably also, said outer surface of the femoral component has a third radius of curvature for varus/valgus rotation in a coronal plane, and the surface of the femoral side of the insert has substantially the same radius of curvature in the coronal plane as the third radius of curvature of the femoral component.

The various aspects of the present invention will now be described by way of example with reference to the accompanying drawings, in which:-
- Fig. 1: is a side view of a knee joint prosthesis including a tibial tray, a femoral component and a floating insert;
- Fig. 2: is a perspective view of a floating insert forming part of a knee joint prosthesis similar to that shown in Fig. 1;
- Figs. 3 and 4: are respectively plan and anterior views of the insert shown in Fig. 2;
- Fig. 5: is a cross-section along the line V-V in Fig. 3;
- Fig. 6: is a cross-section along the line VI-VI in Fig. 3;
- Fig. 7: is a perspective view from above of a tibial tray forming part of the prosthesis shown in Fig. 1.
- Fig. 8: is an anterior elevation of the tibial tray shown in Fig. 7;
- Fig. 9: is a cross-section along the line IX-IX in Fig. 8;
- Figs. 10 to 12: are side views of the knee joint prosthesis similar to that shown in Fig. 1 but with the floating insert shown in cross-section along a sagittal place and with a femoral component of the prosthesis shown at 0°, 30° and 80° of flexion respectively; and
- Fig. 13: is a cross-section along the line XIII-XIII of Fig. 11.

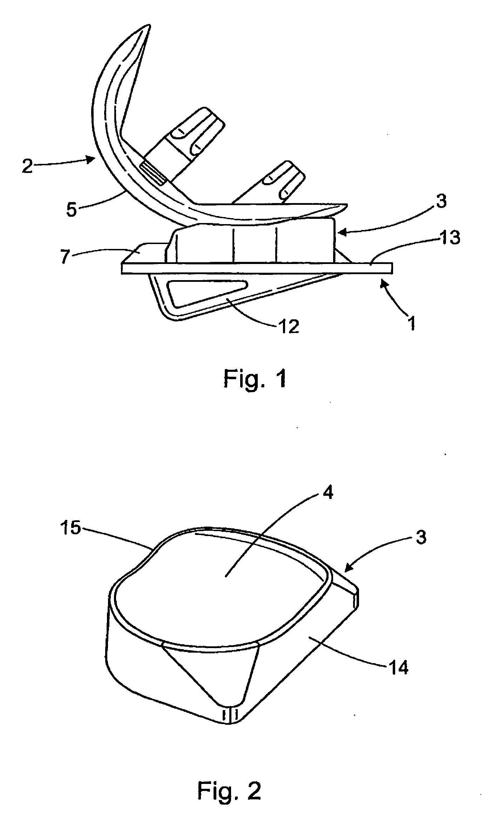

A unicondylar knee joint prosthesis such as is shown in Fig. 1 comprises a tibial tray 1, a femoral component 2 and a floating insert 3. The insert has a femoral side 4 shaped to interface slidingly with an outer surface 5 of the femoral component 2 during flexion of the knee and a tibial side 6 shaped to interface slidingly with a proximal bearing surface 7 of the tibial tray 1. The femoral side 4 of the insert is therefore concave whereas the tibial side 6 of the insert is smooth. In the illustrated embodiment the femoral side 4 is generally planar like the proximal bearing surface 5 of the tibial tray 1 but it could also be convex, concave, curved in a medial-lateral plane and/or curved in an anterior-posterior plane.

Both the tibial tray 1 and the femoral component 2 are preferably made of a material that provides a smooth sliding surface, is sufficiently rigid and durable, and will not cause adverse patient reactions, for example a cobalt chromium molybdenum alloy or oxidized zirconium. In contrast, the floating insert 3 is preferably made of a material that can provide a good sliding interface with both the tibial tray 1 and the femoral component 2 and that also provides good friction and wear characteristics. An example of a suitable material for the insert 3 is ultra high molecular weight polyethylene, a ceramic, oxidized zirconium or a combination of these. The tibial tray 1 and the femoral component 2 are both designed to be cemented in position. Conventional bone cements, such as those based on polymethylmethacrylate can be used.

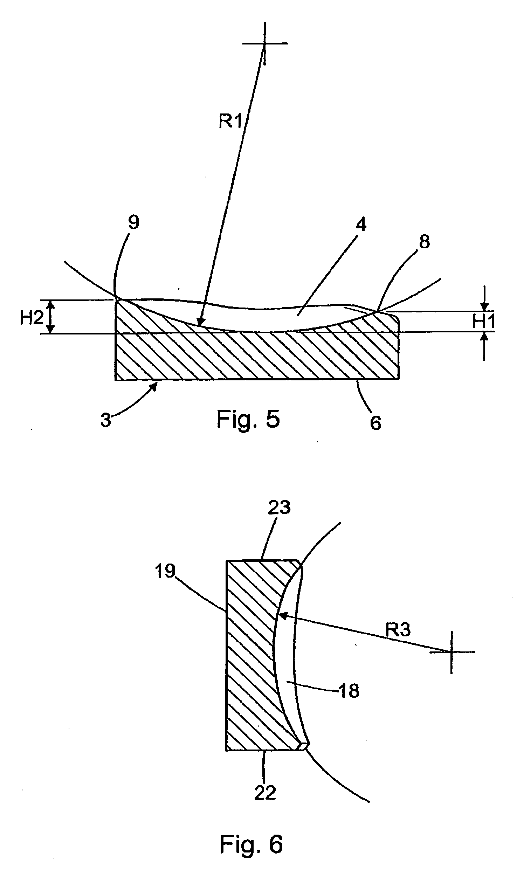

An embodiment of the floating insert for use in such a prosthesis is shown in Figs. 2 to 6. It should be appreciated that the embodiment of knee joint prosthesis and therefore the floating insert shown in Figs. 1 and 11 to 13 of the drawings is shaped for implantation in the lateral side of a left knee or the medial side of a right knee of a patient. A knee joint prostheses suitable for implantation in the medial side of a left knee or the lateral side of a right knee of a patient would be a mirror image of these illustrated prosthesis and the insert 3 for forming part of these prostheses is as shown in Figs. 2 to 6 of the drawings.

During implantation, the floating insert 3 is pushed into position between the femoral component 2 and the tibial tray 1 after these implants have been fitted to a femur and adjacent tibia of a patient. The anterior-posterior profile of the insert 3 is therefore substantially wedge-shaped with the anterior height greater than the posterior height so that it can be pushed into position from an anterior side. The difference in height between the lowest and highest parts of the concave femoral side 4 defines the resistance against luxation of the prosthesis. However, in the sagittal plane the difference H1 in height between the deepest point of the femoral side 4 and a posterior edge 8 of the femoral side 4 should be no greater than around 3 mm and is preferably of the order of 2 mm otherwise too much force is required to push the insert 3 into position between the tibial tray 1 and the femoral component 2. Luxation towards the anterior is prevented by a difference in height H2 between the deepest point of the femoral side 4 of the insert 3 and an anterior edge 9 of the femoral side 4 is between 2mm and 6 mm inclusive and is preferably of the order of 4 mm.

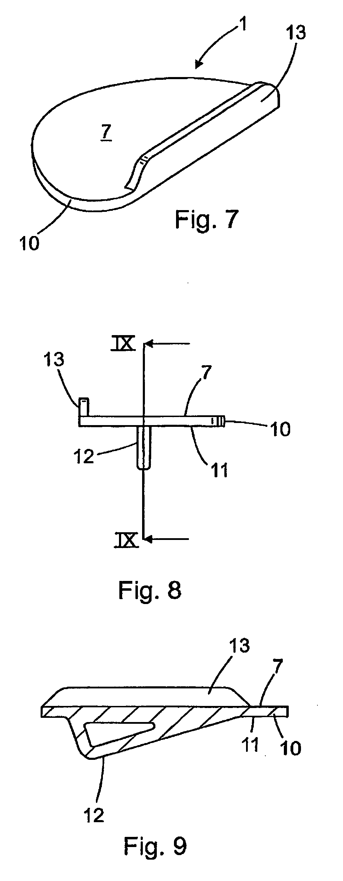

The tibial tray 1 of the knee joint prosthesis is shown in Figs. 7 to 9 of the drawings and comprises a platform 10 defining the proximal bearing surface 7 and a distal fixation surface 11. Projecting from die distal fixation surface 11 is a keel 12 that extends in an anterior-posterior direction with respect to the position of the tray 1 when in use. The keel 12 facilitates positioning and fixation of the tray 1 during implantation to a resected end of a tibia. The proximal bearing surface 7 of the tray 1 is smooth and generally planar so that the tibial side 6 of the insert 3 is also smooth and generally planar. At an intercondylar edge of the platform 10 of the tray 1 is a projecting wall 13 that is linear and that in use extends in a sagittal plane from the proximal bearing surface 7 and projects in a proximal direction. The wall 13 is therefore located on the intercondylar side of the tray 1 and is provided to prevent dislocation of the insert 3 during use and to prevent abrasion of the insert 3 by adjacent bone. In contrast, the superficial, anterior and posterior edges of die platform 10 form a continuous, asymmetric curve which approximates to the exterior profile of die underlying bone so that there is minimal impairment of the function of the cruciate ligaments and leg muscles when the implant is in use.

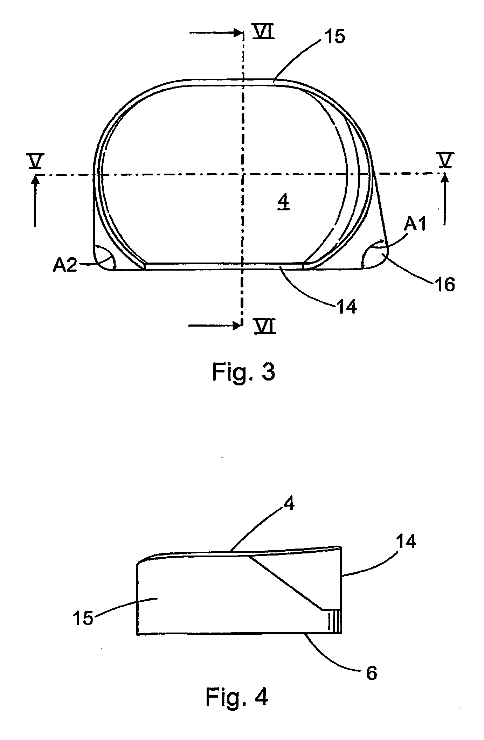

Overall, the insert 3 is asymmetric about its sagittal plane although the surface defined by the femoral side 4 is symmetric about this plane and is approximately rectangular but with rounded corners. Beneath this femoral surface, the insert 3 is shaped to prevent excess rotation relative to the tibial tray 1 from taking place during use and to protect surrounding soft tissues from damage. It has a intercondylar side 14 that extends linearly and that is adapted for location adjacent the wall 13 of the tibial tray 1. This side 14 is longer in length than am opposite superficial side 15 of the insert 3, which has a rounded profile. Contact between the longer intercondylar side 14 of the insert 3 and the wall 13 limits extensive rotation of the insert 3 relative to the tibial tray 1 as the knee joint is moved. In particular, the corner 16 at the posterior end of the intercondylar side 14 may be extended and project rearwardly as shown in Fig. 3 for the express purpose of preventing extensive rotation relating to the tibial tray 1. The angle A1 between die intercondylar side 14 and the posterior side of insert 3 is therefore equal to or less than 90° and is typically around 80°. The corner A2 between the intercondylar side 14 and the anterior side of the insert is around 90° although the actual apexes of both corners are rounded. However, the corners between the superficial side 15 of the insert and its posterior and anterior sides are rounded in order to protect the surrounding soft tissue from damage during deep flexion and during hyperextension of the knee.

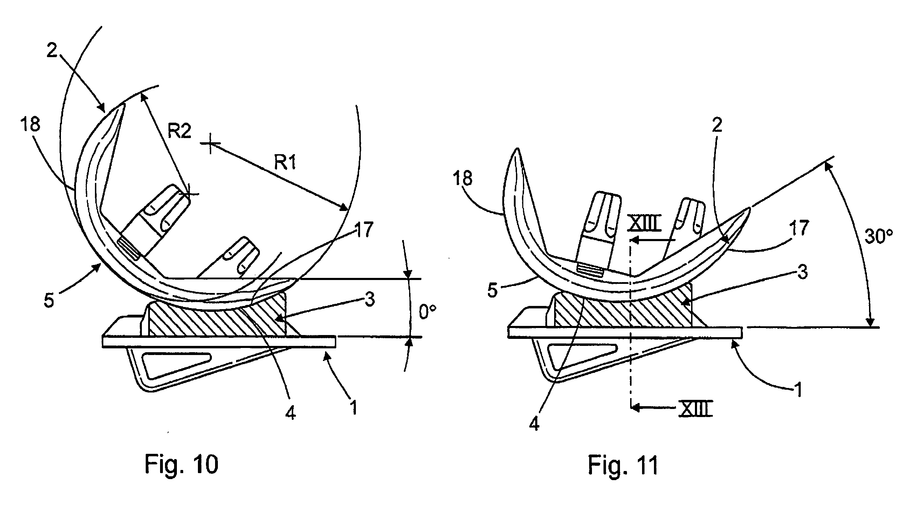

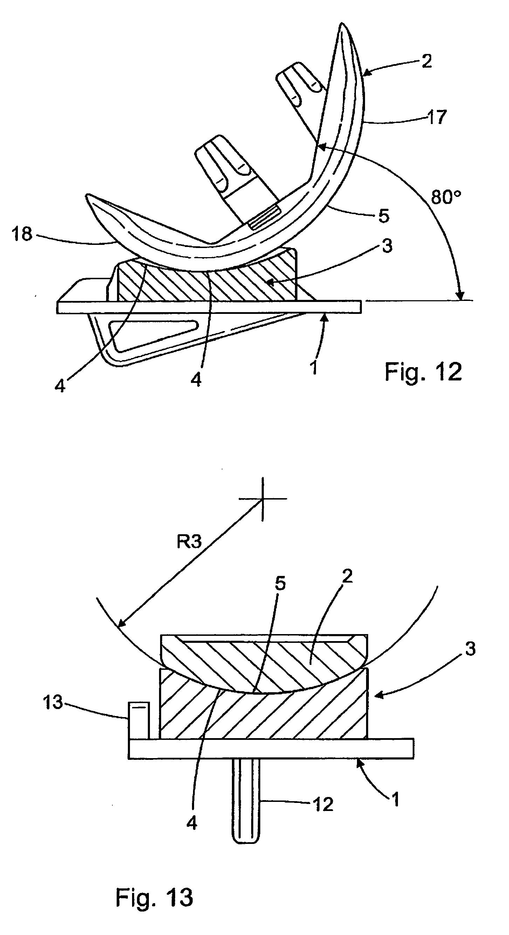

As shown in Fig. 10, in the sagittal plane, the surface 5 of the femoral component 2 defines two regions 17 and 18 for flexion rotation in a sagittal plane of which the first region 16 has a first radius of curvature R1 and die second region 18 has a second radius of curvature R2. The surface 18 with the second radius of curvature R2 defines the posterior part of the component 2 while the surface 17 with the first radius of curvature R1 defines the anterior part of the component 2 and imitates the distal shape of the femoral condyles. The first radius of curvature R1 is larger than the second radius of curvature R2 and typically the ratio of these two radii is around 1.5:1. The surface 5 of the femoral component 2 also has a third radius of curvature R3 in a coronal plane for varus/valgus rotation, as shown in Fig. 13. The radius of curvature R3 is substantially the same as the radius of curvature R2. This means that the surface 18 of the posterior part of the component 2 is spheroidal whereas the surface 17 of the distal or anterior part of the component 2 is toroidal.

As the floating insert 3 is designed to slide between the proximal bearing surface 7 of the tibial tray 1 and the outer surface 5 of the femoral component 2, the concave nature of the femoral side 4 of the insert 3 is such that its surface has substantially the same radius of curvature in the sagittal plane as the first radius of curvature R1 of the distal part of the femoral component 2. This means that there is maximum congruency between the insert 3 and the femoral component 2 in extension. In addition, this surface in the coronal plane has substantially the same radius of curvature as the third radius of curvature R3 of the femoral component 2. This allows varus/valgus rotation of the knee in a coronal plan of up to around 8°.

The generally planar nature of the proximal bearing surface 7 of the tibial tray 1 means that it is not able to guide motion between the femur and the tibia of the patient during movement of the knee joint. This has to be controlled by the patient's own muscles, the capsule and the ligaments around the knee joint. It is therefore important that the prosthesis is implanted with precision in a manner which causes minimum damage to the soft tissues around the knee. The prosthesis is designed to allow a range of motion between around 8° hyper-extension to around 125° flexion. Flexion much beyond 125° depends on the way the bony overhang on the posterior femoral condyle is treated. Preferably, the overhang is removed to prevent impingement between the femur and the insert 3 at greater than 125° of flexion.

During flexion, congruence between the insert 3 and the tibial tray 1 is presumed as the proximal bearing surface 7 and the tibial side 6 of the insert 3 are both generally planar. However, the contact condition between the surface 5 of the femoral component 2 and the femoral side 4 of the insert 3 varies with the degree of flexion. In full extension at 0° of flexion, as shown in Fig. 10, the sagittal and frontal radii are the same so that the surfaces 4 and 5 are totally congruent. This situation is maintained until approximately 30° of flexion when the region 18 with the smaller posterior radius R2 of the surface component 2 comes into contact with the surface 4, as shown in Fig. 11. Between approximately 30° and 80° of flexion, the latter of which is shown in Fig. 12, both regions 17 and 18 of the surface 5 are in contact with the surface 4 of the insert 3 and the contact area between the component 2 and the insert 3 is reduced. Thereafter, beyond approximately 80° only the region 18 of the surface 5 is in contact with the surface 4 of the insert 3 and the contact area between the component 2 and the insert 3 is at its smallest. However, the load between them is at its smallest too. The largest loading between the two components 2 and 3 occurs between 0° and around 30° of flexion when they are both fully congruent.

### Reference Numerals

- 1: Tibial tray
- 2: Femoral component
- 3: Floating insert
- 4: Femoral side of insert
- 5: Outer surface of femoral component
- 6: Tibial side of insert
- 7: Proximal beating surface
- 8: Posterior edge
- 9: Anterior edge
- 10: Platform
- 11: Distal fixation surface
- 12: Keel
- 13: Intercondylar wall
- 14: Intercondylar side
- 15: Superficial side
- 16: Posterior intercondylar corner
- 17: Anterior part of outer surface
- 18: Posterior part of outer surface

- A1: Angle between posterior and intercondylar sides of insert
- A2: Angle between anterior and intercondylar sides of insert
- H1: Posterior height of insert
- H2: Anterior height of insert
- R1: Radius of distal part of femoral component
- R2: Radius of posterior part of femoral component
- R3: Radius in coronal plane

## Claims

1. A floating insert (3) for a unicondylar knee joint prosthesis comprising a femoral component (2) and a tibial tray (1), the insert (3) having a femoral side (4) shaped to interface slidingly with an outer surface (5) of the femoral component (2) during flexion of the knee and a tibial side (6) shaped to interface slidingly with a proximal bearing surface (7) of the tibial tray (1) during flexion of the knee, the insert having an intercondylar side (14) and an opposite superficial side (15) wherein the insert is not symmetrical about a sagittal plane, the intercondylar side (14) being longer in length than the superficial side (15) and **characterised in that** a corner (16) at a posterior end of the intercondylar side (14) is extended to project rearwardly.

2. An insert (3) as claimed in Claim 1, wherein an angle (A1) between the intercondylar side (14) and a posterior side of the insert (3) is equal to or less than 90°.

3. An insert (3) as claimed in Claim 1 or Claim 2, wherein corners between the superficial side (15) of the insert and its posterior and anterior sides are rounded.

4. An insert (3) as claimed in any of Claims 1 to 3, wherein a surface of the tibial side (6) is smooth.

5. An insert (3) as claimed in any of Claims 1 to 4, wherein an anterior-posterior profile is substantially wedge-shaped with an anterior height greater than a posterior height

6. An insert (3) as claimed in any of Claims 1 to 5, wherein in a sagittal plane the difference in height (H1) between a deepest point of the femoral side (4) and a posterior edge (8) of the femoral side (4) is no greater than 3 mm and is preferably of the order of 2mm

7. An insert (3) as claimed in any of Claims 1 to 6, wherein in a sagittal plane the difference in height (H2) between a deepest point of the femoral side (4) and an anterior edge (9) of the femoral side (4) is between 2mm and 6 mm inclusive and is preferably of the order of 4 mm.

8. An insert (3) as claimed in any of claims 1 to 7, wherein it is comprised of one or a combination of an ultra high molecular weight polyethylene, a ceramic or oxidized zirconium.

9. A unicondylar knee joint prosthesis comprising a femoral component (2), a tibial tray (1), and a floating insert (3), the floating insert (3) being as claimed in any of Claims 1 to 8.

10. A prosthesis as claimed in Claim 9, wherein the tibial tray (1) comprises a platform (10) including a proximal bearing surface (7) and a distal fixation surface (11), wherein the proximal bearing surface (7) is smooth.

11. A prosthesis as claimed in Claim 10, wherein the platform (10) of the tibial tray (1) defines a wall (13) that projects in a proximal direction at an intercondylar edge of the proximal bearing surface (7).

12. A prosthesis as claimed in Claim 11, wherein the wall (13) extends proximally, in use, in a sagittal plane.

13. A prosthesis as claimed in any of Claims 9 to 12, wherein an outer surface (5) of the femoral component (2) defines first and second regions (17,18) for flexion rotation in a sagittal plane of which the first region (17) has a first radius of curvature (R1) and the second region (18) has a second radius of curvature (R2), the first radius of curvature (R1) being larger than the second radius of curvature (R2), and the surface of the femoral side (4) of the insert (3) has substantially the same radius of curvature in the sagittal plane as the first radius of curvature (R1) of the femoral component (2).

14. A prosthesis as claimed in any of claims 9 to 13, wherein said outer surface (5) of the femoral component (2) has a third radius of curvature (R3) for varus/valgus rotation in a coronal plane, and the surface of the femoral side (4) of the insert (3) has substantially the same radius of curvature in the coronal plane as the third radius of curvature (R3) of the femoral component (2).

## Patentansprüche

1. Ein beweglicher Einsatz (3) für eine unikondyläre Kniegelenkprothese, die eine Femurkomponente (2) und eine Tibiaplatte (1) beinhaltet, wobei der Einsatz (3) eine Femurseite (4), die geformt ist, um während der Flexion des Knies mit einer äußeren Oberfläche (5) der Femurkomponente (2) gleitend in Verbindung zu sein, und eine Tibiaseite (6), die geformt ist, um während der Flexion des Knies mit einer proximalen Auflagefläche (7) der Tibiaplatte (1) gleitend in Verbindung zu sein, aufweist, wobei der Einsatz eine interkondyläre Seite (14) und eine gegenüberliegende superfizielle Seite (15) aufweist, wobei der Einsatz um eine Sagittalebene nicht symmetrisch ist, die interkondyläre Seite (14) länger als die superfizielle Seite (15) ist, und **dadurch gekennzeichnet, dass** eine Ecke (16) an einem posterioren Ende der interkondylären Seite (14) verlängert ist, um nach hinten vorzustehen.

2. Einsatz (3) gemäß Anspruch 1, wobei ein Winkel (A1) zwischen der interkondylären Seite (14) und einer posterioren Seite des Einsatzes (3) gleich oder kleiner als 90° ist.

3. Einsatz (3) gemäß Anspruch 1 oder Anspruch 2, wobei Ecken zwischen der superfiziellen Seite (15) des Einsatzes und seiner posterioren und anterioren Seite gerundet sind.

4. Einsatz (3) gemäß einem der Ansprüche 1 bis 3, wobei eine Oberfläche der Tibiaseite (6) glatt ist.

5. Einsatz (3) gemäß einem der Ansprüche 1 bis 4, wobei ein anterior-posteriores Profil im Wesentlichen keilförmig ist, wobei eine anteriore Höher größer als eine posteriore Höhe ist.

6. Einsatz (3) gemäß einem der Ansprüche 1 bis 5, wobei in einer Sagittalebene der Unterschied in der Höhe (H1) zwischen einem tiefsten Punkt der Femurseite (4) und einer posterioren Kante (8) der Femurseite (4) nicht mehr als 3 mm beträgt und vorzugsweise ungefähr 2 mm beträgt.

7. Einsatz (3) gemäß einem der Ansprüche 1 bis 6, wobei in einer Sagittalebene der Unterschied in der Höhe (H2) zwischen einem tiefsten Punkt der Femurseite (4) und einer anterioren Kante (9) der Femurseite (4) zwischen 2 mm und 6 mm, einschließlich, beträgt und vorzugsweise ungefähr 4 mm beträgt.

8. Einsatz (3) gemäß einem der Ansprüche 1 bis 7, wobei er aus einem von oder einer Kombination von einem ultrahochmolekularen Polyethylen, einer Keramik oder oxidiertem Zirconium besteht.

9. Eine unikondyläre Kniegelenkprothese, beinhaltend eine Femurkomponente (2), eine Tibiaplatte (1) und einen beweglichen Einsatz (3), wobei der bewegliche Einsatz (3) wie gemäß einem der Ansprüche 1 bis 8 ist.

10. Prothese gemäß Anspruch 9, wobei die Tibiaplatte (1) eine Plattform (10) beinhaltet, die eine proximale Auflagefläche (7) und eine distale Fixierungsoberfläche (11) umfasst, wobei die proximale Auflagefläche (7) glatt ist.

11. Prothese gemäß Anspruch 10, wobei die Plattform (10) der Tibiaplatte (1) eine Wand (13) definiert, die an einer interkondylären Kante der proximalen Auflagefläche (7) in einer proximalen Richtung vorsteht.

12. Prothese gemäß Anspruch 11, wobei sich die Wand (13) in Verwendung in einer Sagittalebene proximal erstreckt.

13. Prothese gemäß einem der Ansprüche 9 bis 12, wobei eine äußere Oberfläche (5) der Femurkomponente (2) einen ersten und zweiten Bereich (17, 18) zur Flexionsrotation in einer Sagittalebene definiert, wovon der erste Bereich (17) einen ersten Krümmungsradius (R1) aufweist und der zweite Bereich (18) einen zweiten Krümmungsradius (R2) aufweist, wobei der ersten Krümmungsradius (R1) größer als der zweite Krümmungsradius (R2) ist, und wobei die Oberfläche der Femurseite (4) des Einsatzes (3) in der Sagittalebene einen Krümmungsradius aufweist, der im Wesentlichen der gleiche wie der erste Krümmungsradius (R1) der Femurkomponente (2) ist.

14. Prothese gemäß einem der Ansprüche 9 bis 13, wobei die äußere Oberfläche (5) der Femurkomponente (2) einen dritten Krümmungsradius (R3) zur Varus/Valgus-Rotation in einer koronaren Ebene aufweist, und wobei die Oberfläche der Femurseite (4) des Einsatzes (3) in der koronaren Ebene einen Krümmungsradius aufweist, der im Wesentlichen der gleiche wie der dritte Krümmungsradius (R3) der Femurkomponente (2) ist.

## Revendications

1. Un insert flottant (3) pour une prothèse d'articulation de genou unicondylienne comprenant un composant fémoral (2) et un plateau tibial (1), l'insert (3) ayant un côté fémoral (4) conformé pour être raccordé de façon à glisser avec une surface externe (5) du composant fémoral (2) durant la flexion du genou et un côté tibial (6) conformé pour être raccordé de façon à glisser avec une surface d'appui proximale (7) du plateau tibial (1) durant la flexion du genou, l'insert ayant un côté intercondylien (14) et un côté superficiel opposé (15), l'insert n'étant pas symétrique autour d'un plan sagittal, le côté intercondylien (14) étant de plus grande longueur que le côté superficiel (15) et étant **caractérisé en ce qu'**un coin (16) au niveau d'une extrémité postérieure du côté intercondylien (14) est étendu pour faire saillie vers l'arrière.

2. Un insert (3) tel que revendiqué dans la revendication 1, dans lequel un angle (A1) entre le côté intercondylien (14) et un côté postérieur de l'insert (3) est égal ou inférieur à 90°.

3. Un insert (3) tel que revendiqué dans la revendication 1 ou la revendication 2, dans lequel des coins entre le côté superficiel (15) de l'insert et ses côtés postérieur et antérieur sont arrondis.

4. Un insert (3) tel que revendiqué dans n'importe lesquelles des revendications 1 à 3, dans lequel une surface du côté tibial (6) est lisse.

5. Un insert (3) tel que revendiqué dans n'importe lesquelles des revendications 1 à 4, dans lequel un profil antérieur-postérieur est substantiellement conformé comme une cale avec une hauteur antérieure supérieure à une hauteur postérieure.

6. Un insert (3) tel que revendiqué dans n'importe lesquelles des revendications 1 à 5, dans lequel, dans un plan sagittal, la différence de hauteur (H1) entre un point le plus profond du côté fémoral (4) et un bord postérieur (8) du côté fémoral (4) n'est pas supérieure à 3 mm et est de préférence de l'ordre de 2 mm.

7. Un insert (3) tel que revendiqué dans n'importe lesquelles des revendications 1 à 6, dans lequel, dans un plan sagittal, la différence de hauteur (H2) entre un point le plus profond du côté fémoral (4) et un bord antérieur (9) du côté fémoral (4) est comprise entre 2 mm et 6 mm inclus et est de préférence de l'ordre de 4 mm.

8. Un insert (3) tel que revendiqué dans n'importe lesquelles des revendications 1 à 7, lequel étant constitué d'un élément ou d'une combinaison de matériaux parmi un polyéthylène à poids moléculaire ultra élevé, une céramique ou un zirconium oxydé.

9. Une prothèse d'articulation de genou unicondylienne comprenant un composant fémoral (2), un plateau tibial (1), et un insert flottant (3), l'insert flottant (3) étant tel que revendiqué dans n'importe lesquelles des revendications 1 à 8.

10. Une prothèse telle que revendiquée dans la revendication 9, dans laquelle le plateau tibial (1) comprend une plateforme (10) incluant une surface d'appui proximale (7) et une surface de fixation distale (11), la surface d'appui proximale (7) étant lisse.

11. Une prothèse telle que revendiquée dans la revendication 10, dans laquelle la plateforme (10) du plateau tibial (1) définit une paroi (13) qui fait saillie dans une direction proximale au niveau d'un bord intercondylien de la surface d'appui proximale (7).

12. Une prothèse telle que revendiquée dans la revendication 11, dans laquelle la paroi (13) s'étend proximalement, lors de l'utilisation, dans un plan sagittal.

13. Une prothèse telle que revendiquée dans n'importe lesquelles des revendications 9 à 12 dans laquelle une surface externe (5) du composant fémoral (2) définit des première et deuxième régions (17, 18) pour la rotation en flexion dans un plan sagittal, la première région (17) ayant un premier rayon de courbure (R1) et la deuxième région (18) ayant un deuxième rayon de courbure (R2), le premier rayon de courbure (R1) étant plus grand que le deuxième rayon de courbure (R2), et la surface du côté fémoral (4) de l'insert (3) ayant substantiellement le même rayon de courbure dans le plan sagittal que le premier rayon de courbure (R1) du composant fémoral (2).

14. Une prothèse telle que revendiquée dans n'importe lesquelles des revendications 9 à 13, dans laquelle ladite surface externe (5) du composant fémoral (2) a un troisième rayon de courbure (R3) pour la rotation varus/valgus dans un plan frontal, et la surface du côté fémoral (4) de l'insert (3) a substantiellement le même rayon de courbure dans le plan frontal que le troisième rayon de courbure (R3) du composant fémoral (2).
